# FASCICULE DE BREVET EUROPEEN

(11) **EP 0 780 114 B1**
(45) Date de publication et mention de la délivrance du brevet: **18.12.2002**
(21) Numéro de dépôt: 96402548.0
(22) Date de dépôt: 26.11.1996
(51) Int. Cl.: A61K 7/00, A61K 7/48

(54) **Nanoémulsion transparente à base de tensioactifs siliconés et utilisation en cosmétique ou en dermopharmacie**
Transparente Nanoemulsion basierend auf silikontensioaktiven Verbindungen und ihre Anwendung in der Kosmetik oder in der Dermopharmazie
Transparent nanoemulsion based on silicon tensio actives and its use in cosmetics or in dermopharmaceutics

(30) Priorité: 21.12.1995 FR 9515291
(43) Date de publication de la demande: 25.06.1997
(73) Titulaire: L'OREAL, 75008 Paris (FR)
(72) Inventeur: Simonnet, Jean-Thierry, 75011 Paris (FR)
(74) Mandataire: Tezier Herman, Béatrice

(56) Documents cités:
- EP-A- 0 226 337
- EP-A- 0 330 369
- EP-A- 0 406 162
- EP-A- 0 407 089
- EP-A- 0 514 934
- EP-A- 0 529 847
- EP-A- 0 532 256
- EP-A- 0 559 013
- EP-A- 0 579 455
- EP-A- 0 615 741
- EP-A- 0 631 774
- EP-A- 0 638 308
- EP-A- 0 674 898
- EP-A- 0 774 482
- FR-A- 2 315 991
- FR-A- 2 597 345
- FR-A- 2 597 367
- FR-A- 2 683 453
- FR-A- 2 693 466
- FR-A- 2 718 019
- JP-A- 7 291 825
- US-A- 4 620 878
- US-A- 5 364 633
- TENSIDE,SURFACTANT,DETERGENTS, vol. 29, no. 2, Mars 1992, MÜNICH, pages 78-83, XP000262468 B. GRÜNING ET AL:

## Description

La présente invention se rapporte à une émulsion huile-dans-eau notamment transparente, dont les globules d'huile ont une taille moyenne inférieure à 100 nm et qui comprend au moins un tensioactif siliconé, ainsi qu'à son utilisation en application topique notamment dans les domaines cosmétique et dermatologique.

Les émulsions huile-dans-eau, où une phase huileuse est dispersée dans une phase aqueuse, sont bien connues dans le domaine de la cosmétique et en dermopharmacie, notamment pour la préparation de produits tels que des lotions, des toniques, des sérums, des crèmes, des eaux de toilette.
Afin d'obtenir des compositions transparentes ayant une apparence proche de l'eau et conduisant, après application sur la peau, à un toucher analogue à celui d'une crème ou d'un lait, on a déjà mis en oeuvre des émulsions comprenant des globules d'huile ayant une taille moyenne inférieure à 100 nm, appelées nanoémulsions.
Ainsi, le document EP-A-406162 décrit des nanoémulsions comprenant une phase lipidique amphiphile constituée de phosphoglycérides. Ces nanoémulsions sont obtenues par un procédé d'homogénéisation à haute pression. Elles présentent l'inconvénient d'être instables au stockage aux températures traditionnelles de conservation à savoir entre 0 et 45°C et de conduire de ce fait à des compositions jaunes, produisant des odeurs de rance qui se développent après quelques jours de conservation.
Par ailleurs, le document EP-A-615741 décrit des nanoémulsions comprenant l'association d'un alcool gras et/ou d'un acide gras à chaîne longue et d'un tensioactif du type savon d'acide gras à chaîne longue, formant un gel de température de transition de phase, supérieure à 60°C. Ces nanoémulsions sont préparées à des températures élevées supérieures à 70°C, ce qui rend impossible l'utilisation d'actifs thermosensibles (par exemple les vitamines) dans de telles compositions.
On connaît également par JP7/291825 des microémulsions de type huile-dans-eau comprenant un organopolysiloxane, une huile pour le dissoudre, une silicone modifiée polyéther, un tensioactif non ionique ou anionique, et de l'eau.
On connaît encore par EP774482 une méthode pour former spontanément des microémulsions stables et claires, comprenant des huiles de silicone, une silicone polyéther et de l'eau.

Il subsiste donc le besoin d'une nanoémulsion ne présentant pas les inconvénients rencontrés avec celles connues jusqu'à présent.
La nanoémulsion selon l'invention permet justement de pallier les problèmes mentionnés précédemment. En effet, la demanderesse a trouvé de manière surprenante qu'il était possible d'obtenir une nanoémulsion stable pouvant être préparée à des températures entre 20 et 45°C, en utilisant un tensioactif particulier.

La présente invention a donc pour objet une émulsion huile-dans-eau comprenant une phase huileuse dispersée dans une phase aqueuse, dont les globules d'huile ont une taille moyenne inférieure à 100 nm, caractérisée en ce qu'elle comprend au moins un tensioactif siliconé.

Les émulsions conformes à l'invention dont les globules d'huile ont une taille moyenne inférieure à 100 nm, sont stables au stockage entre 0 et 45°C après au moins deux mois. Elles sont préparées à des températures entre 20 et 45°C ; il est donc possible d'y introduire des actifs thermosensibles sans que ceux-ci risquent d'être dégradés, et par exemple des vitamines et des huiles végétales contenant des acides gras insaturés.

Par ailleurs, les émulsions selon l'invention peuvent contenir des quantités importantes d'huile tout en conservant de bonnes propriétés de transparence. En outre, elles favorisent la pénétration des actifs dans les couches superficielles de la peau.

Un tensioactif siliconé est un composé siliconé comportant au moins une chaîne oxyéthylénée -OCH₂CH₂- et/ou oxypropylénée -OCH₂CH₂CH₂-. Comme tensioactifs siliconés pouvant être utilisés selon la présente invention, on peut citer ceux décrits dans les documents US-A-5364633 et US-A-5411744.

Le tensioactif siliconé utilisé selon la présente invention est un composé de formule (I) : 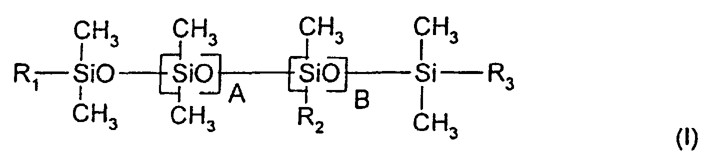 dans laquelle :
R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)_{z} - OR₄, au moins un radical R₁, R₂ ou R₃ n'étant pas un radical alkyle ; R₄ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

Selon un mode de réalisation préféré de l'invention, dans le composé de formule (I), le radical alkyle est un radical méthyle, x est un nombre entier allant de 2 à 6 et y est un nombre entier allant de 4 à 30.

On peut citer, à titre d'exemple de tensioactifs siliconés de formule (I), les composés de formule (II) : 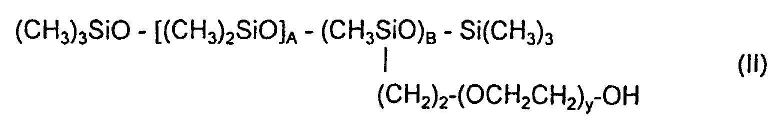 dans laquelle A est un nombre entier allant de 20 à 105, B est un nombre entier allant de 2 à 10 et y est un nombre entier allant de 10 à 20.

On peut également citer à titre d'exemple de tensioactifs siliconés de formule (I), les composés de formule (III) :

HO - (OCH₂CH₂)_{y}-(CH₂)₃ - [(CH₃)₂SiO]_{A'} - (CH₂)₃ - (OCH₂CH₂)_{y} - OH (III)

dans laquelle A' et y sont des nombres entiers allant de 10 à 20.

On peut utiliser comme composés de l'invention ceux vendus par la société Dow Corning sous les dénominations DC 5329, DC 7439-146, DC 2-5695 et Q4-3667. Les composés DC 5329, DC 7439-146, DC 2-5695 sont des composés de formule (II) où respectivement A est 22, B est 2 et y est 12 ; A est 103, B est 10 et y est 12 ; A est 27, B est 3 et y est 12.

Le composé Q4-3667 est un composé de formule (III) où A est 15 et y est 13.

Le rapport en poids de la quantité de phase huileuse contenue dans l'émulsion selon l'invention sur la quantité de tensioactif siliconé varie de 2 à 10 et plus préférentiellement de 3 à 6.

La quantité de tensioactif siliconé dans l'émulsion selon l'invention va, de préférence, de 1 à 15 % en poids, et mieux de 3 à 6 % en poids par rapport au poids total de l'émulsion.

L'émulsion conforme à l'invention comporte une quantité de phase huileuse allant, de préférence, de 5 à 40 % en poids et mieux 10 à 30 % en poids par rapport au poids total de l'émulsion.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles essentielles naturelles ou synthétiques (huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote), les huiles siliconées volatiles (décaméthylcyclopentasiloxane) ou non volatiles (dodécaméthylcyclohexasiloxane), les huiles fluorées (perfluoropolyéthers), les carbures halogénés (fluorocarbures tels que des fluoramines par exemple la perfluorotributylamine, des hydrocarbures fluorés, par exemple le perfluorodécahydronaphtalène). On peut aussi utiliser comme matières grasses des alcools gras, des acides gras, des cires, des gommes (gomme de silicone).

L'émulsion conforme à l'invention contient en plus un ou plusieurs lipides amphiphiles ioniques.

Les lipides amphiphiles ioniques utilisés dans les nanoémulsions de l'invention, sont choisis, de préférence, dans le groupe formé par les lipides anioniques neutralisés, les lipides ioniques amphotères, les dérivés alkylsulfoniques.

Ils sont choisis dans le groupe formé par :
- les sels alcalins du dicetyl- et du dimyristylphosphate ;
- les sels alcalins du cholestérol sulfate ;
- les sels alcalins du cholestérol phosphate ;
- les lipoaminoacides tels que les acylglutamates mono et disodiques ;
- les sels de sodium de l'acide phosphatidique ;
- les phospholipides ;
- les dérivés alkylsulfoniques de formule : 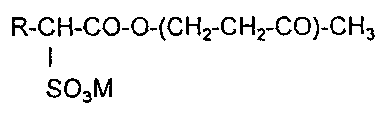 dans laquelle R représente des radicaux alkyle en C₁₆-C₂₂, en particulier les radicaux C₁₆H₃₃ et C₁₈H₃₇, pris en mélange ou séparément, et M est un métal alcalin tel que le sodium et le potassium ;
- et leurs mélanges.

Comme lipide amphiphile ionique, on peut citer en particulier le sel monosodique de l'acide N-stéaroylglutamique vendu sous la dénomination « acylglutamate HS 21 » par la société Ajinomoto, le dicétylphosphate de sodium et le dimyristylphosphate de sodium.

Les lipides ioniques amphiphiles sont en particulier présents à des concentrations allant de 0 à 20 % en poids, plus particulièrement de 5 à 15 % en poids par rapport au poids de tensioactif siliconé.

L'émulsion conforme à la présente invention peut contenir des additifs pour améliorer la transparence de la formulation.

Ces additifs peuvent être choisis par exemple dans le groupe formé par :
- les alcools inférieurs en C₁-C₈ tels que l'éthanol ;
- les glycols tels que la glycérine, le propylèneglycol, le 1,3-butylèneglycol, le dipropylèneglycol, les polyéthylèneglycols comportant de 4 à 16 unités d'oxyde d'éthylène et de préférence de 8 à 12 ;
- et leurs mélanges.

Les additifs tels que ceux cités ci-dessus sont utilisés dans les émulsions de l'invention de préférence à des concentrations allant de 0 à 30 % en poids et de préférence en une concentration supérieure à 5 % en poids par rapport au poids total de l'émulsion. Les alcools sont utilisés, de préférence, à des concentrations allant de 5 à 20 % en poids par rapport au poids total de l'émulsion, tandis que les glycols sont utilisés, de préférence, à des concentrations allant de 5 à 15 % en poids.

En outre, l'utilisation des alcools tels que définis ci- dessus, à des concentrations supérieures ou égales à 15 % en poids permet d'obtenir des émulsions sans conservateur.

Les globules d'huile des émulsions de l'invention, ont de préférence une taille moyenne allant de 15 à 100 nm, et mieux de 15 à 90 nm. La diminution de la taille des globules permet de favoriser la pénétration des actifs dans les couches superficielles de la peau (effet véhicule) et d'améliorer la transparence de l'émulsion.

Les émulsions selon l'invention sont incolores et éventuellement légèrement bleutées et présentent une transparence déterminée par le coefficient de transmittance mesuré à une longueur d'onde de 600 nm, allant, préférentiellement, de 30 à 90 % et plus particulièrement de 50 à 80 %.

Le procédé pour obtenir les émulsions selon l'invention comprend les étapes suivantes :
- on mélange la phase aqueuse et la phase huileuse, sous agitation vive, à une température ambiante inférieure à 45°C ;
- on effectue une homogénéisation haute pression à une pression supérieure à 10⁸ Pa et de préférence allant de 12.10⁷ à 18.10⁷ Pa.

Un tel procédé permet de réaliser, à température ambiante, des nanoémulsions compatibles avec des composés actifs thermosensibles. Par ailleurs, ces nanoémulsions peuvent contenir des quantités importantes d'huiles et notamment des parfums qui renferment des corps gras, sans les dénaturer.

Pour une application topique, l'émulsion selon l'invention contient, en outre, avantageusement un milieu cosmétiquement et/ou dermatologiquement et/ou pharmaceutiquement acceptable.

L'invention a aussi pour objet l'utilisation de l'émulsion définie ci-dessus pour le traitement cosmétique de la peau et/ou des muqueuses et/ou des ongles et/ou du cuir chevelu et/ou des cheveux ainsi que pour la préparation d'une composition destinée au traitement dermatologique des maladies de la peau et/ou des muqueuses et/ou des ongles et/ou du cuir chevelu et/ou des cheveux.

L'invention a encore pour objet un procédé de traitement thérapeutique et/ou non-thérapeutique de la peau et/ou des muqueuses et/ou des ongles et/ou du cuir chevelu et/ou des cheveux consistant à appliquer l'émulsion définie ci-dessus sur la peau et/ou les muqueuses et/ou les ongles et/ou le cuir chevelu et/ou les cheveux.

L'émulsion permet en particulier une bonne hydratation de la peau, le traitement des peaux grasses et des peaux sensibles. Aussi, la présente invention se rapporte également à l'utilisation cosmétique de l'émulsion définie ci-dessus pour hydrater la peau et/ou traiter la peau grasse et/ou traiter les peaux sensibles. Pour la définition des peaux sensibles, on peut se référer au document EP-A-680749.

L'émulsion selon l'invention peut se présenter notamment sous forme de lotion, de sérum, de gel et contenir les adjuvants habituellement utilisés dans les domaines considérés, tels que des gélifiants, des conservateurs, des antioxydants, des parfums, des charges, des matières colorantes et des vésicules lipidiques.

L'émulsion selon l'invention peut notamment constituer des compositions de nettoyage, de protection, de traitement ou de soin pour le visage, pour le cou, pour les mains, pour les ongles ou pour le corps (par exemple lait de nettoyage, lait de démaquillage, lait corporel), des compositions de bronzage artificiel, ou des compositions pour le bain ou la douche. L'émulsion selon l'invention peut constituer aussi des shampooings, des lotions de mise en plis, des lotions traitantes, des crèmes ou des gels coiffants, des compositions de teintures, des lotions ou des gels antichute.

L'émulsion selon l'invention peut contenir des actifs hydrosolubles ou liposolubles, ayant une activité cosmétique ou dermatologique. Les actifs liposolubles sont dans les globules huileux de l'émulsion, tandis que les actifs hydrosolubles sont dans la phase aqueuse de l'émulsion. On peut citer par exemple comme actif liposoluble le rétinol (vitamine A) et ses dérivés tels que le palmitate de rétinol.

Les exemples qui suivent, permettront de mieux comprendre l'invention, sans toutefois présenter un caractère limitatif.

Pour ces exemples, le mode opératoire suivant est mis en oeuvre :
- dans une première phase A, on homogénéise le mélange des constituants à une température de 45°C ;
- dans une seconde phase B, on dissout les actifs et adjuvants hydrophiles à une température de 20 à 30°C ;
- puis on mélange les phases A et B à l'aide d'un homogénéisateur à turbine et ensuite on homogénéise à l'aide d'un homogénéisateur à haute pression du type Soavi-Niro à une pression de 1500 bars, en 7 passages en maintenant la température du produit en dessous de 30°C.

Si l'éthanol est présent dans la composition, il est ajouté à la phase A juste avant addition de celle-ci à la phase B.

### Exemple 1 : Fluide de soin de jour

### Première phase :

- Tensioactif siliconé (DC 2-5695) 4,5 %
- Sel disodique de l'acide N-stéaroyl L-glutamique commercialisé sous la dénomination Acylglutamate HS21 par la Société Ajinomoto (lipide amphiphile ionique) 0,5 %
- Cyclométhicone 6 %
- Huile de jojoba 6 %
- Huile d'avocat 3 %
- Palmitate de rétinol 0,3 %
- Ethanol absolu non dénaturé 15 %

### Deuxième phase :

- Glycérine 5 %
- Eau déminéralisée qsp 100 %

On obtient une émulsion transparente dont la taille des globules est de 57 nm et la transparence de 67 %.

Cette émulsion fluide est apte à traiter la peau.

## Revendications

1. Emulsion huile-dans-eau comprenant une phase huileuse dispersée dans une phase aqueuse, dont les globules d'huile ont une taille moyenne inférieure à 100 nm, **caractérisée en ce qu'**elle comprend au moins un tensioactif siliconé de formule (I): 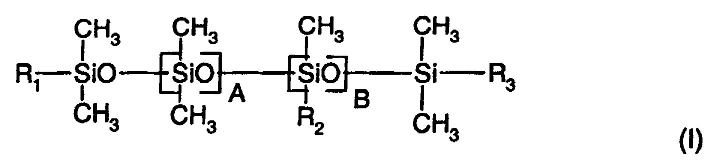 dans laquelle
R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)_{z} - OR₄, au moins un radical R₁, R₂ ou R₃ n'étant pas un radical alkyle ; R₄ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5;
**en ce que** le rapport en poids de la quantité de phase huileuse sur la quantité de tensioactif siliconé varie de 2 à 10,
et **en ce qu'**elle contient, en outre, au moins un lipide amphiphile ionique choisi dans le groupe formé par :
- les sels alcalins du dicetyl- et du dimyristylphosphate ;
- les sels alcalins du cholestérol sulfate ;
- les sels alcalins du cholestérol phosphate ;
- les sels de lipoaminoacides ;
- les sels de sodium de l'acide phosphatidique ;
- les phospholipides ;
- les dérivés alkylsulfoniques de formule : R-CH(SO₃M)-CO-O-(CH₂-CH₂-CO)-CH₃ dans laquelle R représente des radicaux alkyle en C₁₆-C₂₂, pris en mélange ou séparément et M est un métal alcalin,
- et leurs mélanges.

2. Emulsion selon la revendication 1, **caractérisée en ce que** le tensioactif siliconé est un composé de formule (I) où le radical alkyle est un radical méthyle, x est un nombre entier allant de 2 à 6 et y est un nombre entier allant de 4 à 30.

3. Emulsion selon l'une quelconque des revendications précédentes, **caractérisée en ce que** le tensioactif siliconé est un composé de formule (II) : 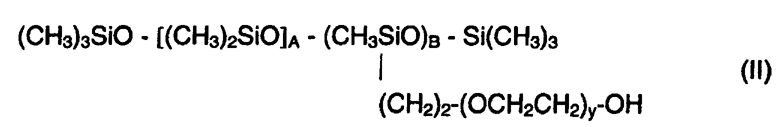 dans laquelle A est un nombre entier allant de 20 à 105, B est un nombre entier allant de 2 à 10 et y est un nombre entier allant de 10 à 20.

4. Emulsion selon la revendication précédente, **caractérisée en ce que** le tensioactif siliconé est choisi parmi les composés de formule (II) où A est 22, B est 2 et y est 12 ; A est 103, B est 10 et y est 12 ; A est 27, B est 3 et y est 12.

5. Emulsion selon l'une quelconque des revendications précédentes, **caractérisée en ce que** le rapport en poids de la quantité de phase huileuse sur la quantité de tensioactif siliconé varie de 3 à 6.

6. Emulsion selon l'une quelconque des revendications précédentes, **caractérisée en ce que** la quantité de tensioactif siliconé va de 1 à 15% en poids par rapport au poids total de l'émulsion.

7. Emulsion selon l'une quelconque des revendications précédentes, **caractérisée en ce que** la quantité de tensioactif siliconé va de 3 à 6% en poids par rapport au poids total de l'émulsion.

8. Emulsion selon l'une quelconque des revendications précédentes, **caractérisée en ce que** la phase huileuse est présente en une quantité allant de 5 à 40% en poids par rapport au poids total de l'émulsion.

9. Emulsion selon l'une quelconque des revendications précédentes, **caractérisée en ce que** la phase huileuse est présente en une quantité allant de 10 à 30 % en poids par rapport au poids total de l'émulsion.

10. Emulsion selon l'une quelconque des revendications précédentes, **caractérisée en ce que** le lipide amphiphile ionique est présent en des concentrations allant de 5 à 15 % en poids par rapport au poids de tensioactif siliconé.

11. Emulsion selon l'une quelconque des revendications précédentes, **caractérisée en ce que** la phase huileuse comprend au moins une huile siliconée et/ou une gomme siliconée

12. Emulsion selon l'une quelconque des revendications précédentes, **caractérisée en ce que** la taille des particules est telle que l'émulsion est transparente.

13. Emulsion selon l'une quelconque des revendications précédentes, **caractérisée en ce qu'**elle contient un additif permettant d'améliorer la transparence.

14. Emulsion selon la revendication précédente, **caractérisée en ce que** l'additif est choisi parmi les alcools inférieurs, les glycols et leurs mélanges.

15. Emulsion selon l'une des revendications 13 à 14, **caractérisée en ce que** l'additif est présent dans des concentrations allant de 5 à 30 % en poids par rapport au poids total de l'émulsion.

16. Emulsion selon l'une quelconque des revendications précédentes, **caractérisée en ce que** les globules d'huile ont une taille moyenne allant de 15 à 90 nm.

17. Emulsion selon l'une quelconque des revendications précédentes, **caractérisée en ce qu'**elle consiste en une composition cosmétique et/ou dermatologique.

18. Emulsion selon l'une quelconque des revendications précédentes, **caractérisée en ce qu'**elle contient un actif cosmétique ou dermatologique, hydrosoluble ou liposoluble.

19. Utilisation de l'émulsion selon l'une quelconque des revendications précédentes pour le traitement cosmétique de la peau et/ou des muqueuses et/ou des ongles et/ou du cuir chevelu et/ou des cheveux.

20. Utilisation de l'émulsion selon l'une quelconque des revendications 1 à 18 pour la préparation d'une composition destinée au traitement dermatologique des maladies de la peau et/ou des muqueuses et/ou des ongles et/ou du cuir chevelu et/ou des cheveux.

21. Utilisation cosmétique de l'émulsion selon l'une quelconque des revendications 1 à 18 pour hydrater la peau et/ou traiter la peau grasse et/ou traiter les peaux sensibles.

22. Procédé de traitement non-thérapeutique de la peau et/ou des muqueuses et/ou des ongles et/ou du cuir chevelu et/ou des cheveux, **caractérisé en ce qu'**il consiste à appliquer sur la peau et/ou les muqueuses et/ou les ongles et/ou le cuir chevelu et/ou les cheveux, l'émulsion selon l'une quelconque des revendications 1 à 18.

## Claims

1. Oil-in-water emulsion comprising an oily phase dispersed in an aqueous phase, the oil globules of which have a mean size of less than 100 nm, **characterized in that** it comprises at least one silicone surfactant of formula (I): 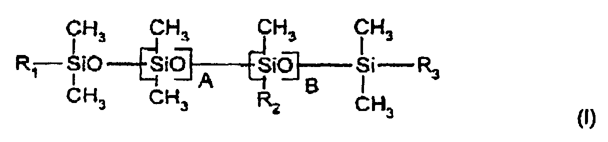 in which:
R₁, R₂ and R₃, independently of one another, represent a C₁-C₆ alkyl radical or a -(CH₂)ₓ-(OCH₂CH₂)_{y}-(OCH₂CH₂CH₂)_{z}-OR₄ radical, at least one R₁, R₂ or R₃ radical not being an alkyl radical; R₄ being a hydrogen, an alkyl radical or an acyl radical;
A is an integer ranging from 0 to 200;
B is an integer ranging from 0 to 50; provided that A and B are not simultaneously zero;
x is an integer ranging from 1 to 6;
y is an integer ranging from 1 to 30;
z is an integer ranging from 0 to 5;
**in that** the ratio by weight of the amount of oily phase to the amount of silicone surfactant varies from 2 to 10,
and **in that** it additionally contains at least one ionic amphiphilic lipid chosen from the group formed by:
- alkaline salts of dicetyl and dimyristyl phosphate;
- alkaline salts of cholesterol sulphate;
- alkaline salts of cholesterol phosphate;
- salts of lipoamino acids;
- sodium salts of phosphatidic acid;
- phospholipids;
- alkylsulphonic derivatives of formula: R-CH(SO₃M)-CO-O-(CH₂-CH₂-CO)-CH₃
in which R represents C₁₆-C₂₂ alkyl radicals, taken as a mixture or separately, and M is an alkali metal,
- and their mixtures.

2. Emulsion according to Claim 1, **characterized in that** the silicone surfactant is a compound of formula (I) where the alkyl radical is a methyl radical, x is an integer ranging from 2 to 6 and y is an integer ranging from 4 to 30.

3. Emulsion according to either one of the preceding claims, **characterized in that** the silicone surfactant is a compound of formula (II) : 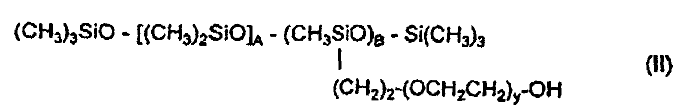 in which A is an integer ranging from 20 to 105, B is an integer ranging from 2 to 10 and y is an integer ranging from 10 to 20.

4. Emulsion according to the preceding claim, **characterized in that** the silicone surfactant is chosen from the compounds of formula (II) where A is 22, B is 2 and y is 12; A is 103, B is 10 and y is 12; and A is 27, B is 3 and y is 12.

5. Emulsion according to any one of the preceding claims, **characterized in that** the ratio by weight of the amount of oily phase to the amount of silicone surfactant varies from 3 to 6.

6. Emulsion according to any one of the preceding claims, **characterized in that** the amount of silicone surfactant ranges from 1 to 15% by weight with respect to the total weight of the emulsion.

7. Emulsion according to any one of the preceding claims, **characterized in that** the amount of silicone surfactant ranges from 3 to 6% by weight with respect to the total weight of the emulsion.

8. Emulsion according to any one of the preceding claims, **characterized in that** the oily phase is present in an amount ranging from 5 to 40% by weight with respect to the total weight of the emulsion.

9. Emulsion according to any one of the preceding claims, **characterized in that** the oily phase is present in an amount ranging from 10 to 30% by weight with respect to the total weight of the emulsion.

10. Emulsion according to any one of the preceding claims, **characterized in that** the ionic amphiphilic lipid is present in concentrations ranging from 5 to 15% by weight with respect to the weight of silicone surfactant.

11. Emulsion according to any one of the preceding claims, **characterized in that** the oily phase comprises at least one silicone oil and/or one silicone gum.

12. Emulsion according to any one of the preceding claims, **characterized in that** the size of the particles is such that the emulsion is transparent.

13. Emulsion according to any one of the preceding claims, **characterized in that** it contains an additive which makes it possible to improve the transparency.

14. Emulsion according to the preceding claim, **characterized in that** the additive is chosen from lower alcohols, glycols and their mixtures.

15. Emulsion according to either of Claims 13 to 14, **characterized in that** the additive is present in concentrations ranging from 5 to 30% by weight with respect to the total weight of the emulsion.

16. Emulsion according to any one of the preceding claims, **characterized in that** the oil globules have a mean size ranging from 15 to 90 nm.

17. Emulsion according to any one of the preceding claims, **characterized in that** it comprises a cosmetic and/or dermatological composition.

18. Emulsion according to any one of the preceding claims, **characterized in that** it contains a watersoluble or fat-soluble cosmetic or dermatological active principle.

19. Use of the emulsion according to any one of the preceding claims for the cosmetic treatment of the skin and/or mucous membranes and/or nails and/or scalp and/or hair.

20. Use of the emulsion according to any one of Claims 1 to 18 for the preparation of a composition intended for the dermatological treatment of diseases of the skin and/or mucous membranes and/or nails and/or scalp and/or hair.

21. Cosmetic use of the emulsion according to any one of Claims 1 to 18 for moisturizing the skin and/or treating greasy skin and/or treating sensitive skin.

22. Process for the non-therapeutic treatment of the skin and/or mucous membranes and/or nails and/or scalp and/or hair, **characterized in that** it comprises the application to the skin and/or mucous membranes and/or nails and/or scalp and/or hair of the emulsion according to any one of Claims 1 to 18.

## Patentansprüche

1. Öl-in-Wasser-Emulsion, die eine in einer wäßrigen Phase dispergierte Ölphase enthält, deren Ölkügelchen eine mittlere Größe unter 100 nm aufweisen, **dadurch gekennzeichnet, daß** sie mindestens einen siliconhaltigen grenzflächenaktiven Stoff der Formel (I) enthält: 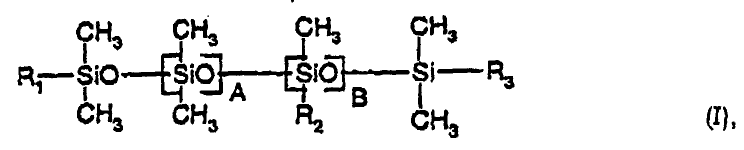 worin bedeuten:
- R₁, R₂ und R₃ unabhängig voneinander eine C₁₋₆-Alkylgruppe oder eine Gruppe -(CH₂)ₓ-(OCH₂CH₂)_{y}-(OCH₂CH₂CH₂)_{z}-OR₄, wobei mindestens eine der Gruppen R₁, R₂ oder R₃ keine Alkylgruppe ist und R₄ Wasserstoff, eine Alkylgruppe oder eine Acylgruppe bedeutet,
- A Null oder eine gänze Zahl von 1 bis 200,
- B Null oder eine ganze Zahl von 1 bis 50, mit der Maßgabe, daß A und B nicht gleichzeitig Null bedeuten,
- x eine ganze Zahl von 1 bis 6,
- y eine ganze Zahl von 1 bis 30,
und
- z Null oder eine ganze Zahl von 1 bis 5,
dadurch, daß das Gewichtsverhältnis der Menge der Ölphase und der Menge des grenzflächenaktiven Silicons im Bereich von 2 bis 10 liegt,
und dadurch, daß sie ferner mindestens ein ionisches amphiphiles Lipid enthält, das ausgewählt ist unter:
- den Alkalisalzen von Dicetyl- und Dimyristylphosphat;
- den Alkalisalzen von Cholesterinsulfat;
- den Alkalisalzen von Cholesterinphosphat;
- den Lipoaminosäuren;
- den Natriumsalzen von Phosphatidsäure;
- den Phospholipiden;
- den Alkylsulfonsäurederivaten der Formel:
R-CH(SO₃M)-CO-O-(CH₂-CH₂-CO)-CH₃, in der R eine Alkylgruppe mit 16 bis 22 Kohlenstoffatomen als Gemisch oder einzeln bedeutet und M ein Alkalimetall ist; und
- deren Gemischen.

2. Emulsion nach Anspruch 1, **dadurch gekennzeichnet, daß** der siliconhaltige grenzflächenaktive Stoff eine Verbindung der Formel (I) ist, in der die Alkylgruppe eine Methylgruppe, x eine ganze Zahl von 2 bis 6 und y eine ganze Zahl von 4 bis 30 bedeutet.

3. Emulsion nach einem der vorhergehenden Ansprüche, **dadurch gekennzeichnet, daß** der siliconhaltige grenzflächenaktive Stoff eine Verbindung der Formel (II) ist: 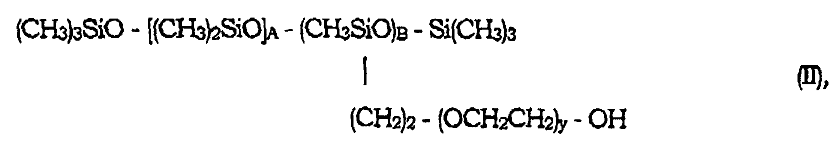 worin bedeuten: A eine ganze Zahl von 20 bis 105, B eine ganze Zahl von 2 bis 10 und y eine ganze Zahl von 10 bis 20.

4. Emulsion nach dem vorhergehenden Anspruch, **dadurch gekennzeichnet, daß** der siliconhaltige grenzflächenaktive Stoff unter den Verbindungen der Formel (II) ausgewählt ist, in der A 22, B 2 und y 12 bzw. A 103, B 10 und y 12 bzw. A 27, B 3 und y 12 bedeuten.

5. Emulsion nach einem der vorhergehenden Ansprüche, **dadurch gekennzeichnet, daß** das Gewichtsverhältnis der Menge der Ölphase und der Menge des grenzflächenaktiven Silicons im Bereich von 3 bis 6 liegt.

6. Emulsion nach einem der vorhergehenden Ansprüche, **dadurch gekennzeichnet, daß** der siliconhaltige grenzflächenaktive Stoff in einem Mengenanteil von 1 bis 15 Gew.-%, bezogen auf das Gesamtgewicht der Emulsion, vorliegt.

7. Emulsion nach einem der vorhergehenden Ansprüche, **dadurch gekennzeichnet, daß** der siliconhaltige grenzflächenaktive Stoff in einem Mengenanteil von 3 bis 6 Gew.-%, bezogen auf das Gesamtgewicht der Emulsion, vorliegt.

8. Emulsion nach einem der vorhergehenden Ansprüche, **dadurch gekennzeichnet, daß** die Ölphase in einem Mengenanteil von 5 bis 40 Gew.-%, bezogen auf das Gesamtgewicht der Emulsion, vorliegt.

9. Emulsion nach einem der vorhergehenden Ansprüche, **dadurch gekennzeichnet, daß** die Ölphase in einem Mengenanteil von 10 bis 30 Gew.-%, bezogen auf das Gesamtgewicht der Emulsion, vorliegt.

10. Emulsion nach einem der vorhergehenden Ansprüche, **dadurch gekennzeichnet, daß** das anionische amphiphile Lipid in Konzentrationen von 5 bis 15 Gew.-%, bezogen auf das Gesamtgewicht des siliconhaltigen grenzflächenaktiven Stoffes, vorliegt.

11. Emulsion nach einem der vorhergehenden Ansprüche, **dadurch gekennzeichnet, daß** die Ölphase mindestens ein Siliconöl und/oder einen Silicongummi enthält.

12. Emulsion nach einem der vorhergehenden Ansprüche, **dadurch gekennzeichnet, daß** die Größe der Partikel so ist, daß die Emulsion transparent ist.

13. Emulsion nach einem der vorhergehenden Ansprüche, **dadurch gekennzeichnet, daß** sie einen Zusatzstoff enthält, mit dem die Transparenz verbessert werden kann.

14. Emulsion nach dem vorhergehenden Anspruch, **dadurch gekennzeichnet, daß** der Zusatzstoff unter den niederen Alkoholen, Glykolen und deren Gemischen ausgewählt ist.

15. Emulsion nach einem der Ansprüche 13 bis 14, **dadurch gekennzeichnet, daß** der Zusatzstoff in Konzentrationen von 5 bis 30 Gew.-%, bezogen auf das Gesamtgewicht der Emulsion, vorliegt.

16. Emulsion nach einem der vorhergehenden Ansprüche, **dadurch gekennzeichnet, daß** die Ölkügelchen eine mittlere Größe von 15 bis 90 nm aufweisen.

17. Emulsion nach einem der vorhergehenden Ansprüche, **dadurch gekennzeichnet, daß** sie eine kosmetische und/oder dermatologische Zusammensetzung ist.

18. Emulsion nach einem der vorhergehenden Ansprüche, **dadurch gekennzeichnet, daß** sie ein wasserlöslichen oder fettlöslichen, kosmetischen oder dermatologischen Wirkstoff enthält.

19. Verwendung der Emulsion nach einem der vorhergehenden Ansprüche zur kosmetischen Behandlung der Haut und/oder der Schleimhäute und/oder der Nägel und/oder der Kopfhaut und/oder der Haare.

20. Verwendung der Emulsion nach einem der Ansprüche 1 bis 18 zur Herstellung einer Zusammensetzung, die zur dermatologischen Behandlung von Erkrankungen der Haut und/oder der Schleimhäute und/oder der Nägel und/oder der Kopfhaut und/oder der Haare vorgesehen ist.

21. Kosmetische Verwendung der Emulsion nach einem der Ansprüche 1 bis 18 zur Hydratisierung der Haut und/oder Behandlung von fettiger Haut und/oder Behandlung von empfindlicher Haut.

22. Verfahren zur nicht therapeutischen Behandlung der Haut und/oder der Schleimhäute und/oder der Nägel und/oder der Kopfhaut und/oder der Haare, **dadurch gekennzeichnet, daß** auf die Haut und/oder die Schleimhäute und/oder die Nägel und/oder die Kopfhaut und/oder die Haare eine Emulsion nach einem der Ansprüche 1 bis 18 aufgetragen wird.
